# EUROPÄISCHE PATENTSCHRIFT

(11) **EP 0 815 072 B1**
(45) Veröffentlichungstag und Bekanntmachung des Hinweises auf die Patenterteilung: **28.06.2000**
(21) Anmeldenummer: 96902993.3
(22) Anmeldetag: 21.02.1996
(51) Int. Cl.: C07C 237/46, A61K 49/00, A61K 49/04

(54) **RÖNTGENKONTRASTMITTEL FÜR DIE COMPUTERTOMOGRAPHIE UND UROGRAPHIE**
RADIOGRAPHIC CONTRASTING AGENT FOR COMPUTERISED TOMOGRAPHY AND UROGRAPHY
PRODUIT DE CONTRASTE RADIOGRAPHIQUE POUR TOMODENSITOMETRIE ET UROGRAPHIE

(30) Priorität: 16.03.1995 DE 19510864
(43) Veröffentlichungstag der Anmeldung: 07.01.1998
(73) Patentinhaber: SCHERING AKTIENGESELLSCHAFT, 13342 Berlin (DE)
(72) Erfinder: BLASZKIEWICZ, Peter, D-12167 Berlin (DE); SPECK, Ulrich, D-13465 Berlin (DE); KRAUSE, Werner, D-13505 Berlin (DE)
(86) Internationale Anmeldenummer: EP9600735
(87) Internationale Veröffentlichungsnummer: WO9628415

(56) Entgegenhaltungen:
- EP-A- 0 015 867
- EP-A- 0 426 610
- INVEST. RADIOL. (1994), 29(1), 72-80, XP000570656 KRAUSE, WERNER ET AL: "Physicochemical parameters of x-ray contrast media"

## Beschreibung

Die Erfindung betrifft den in den Patentansprüchen gekennzeichneten Gegenstand, das heißt neue jodhaltige Röntgenkontrastmittel, deren Herstellung und Verwendungen. Die neuen Verbindungen lösen aktuelle Probleme der kontrastverstärkten Computertomographie. Darüber hinaus sind sie hervorragend für die Urographie geeignet.

Für die medizinische Röntgendiagnostik sind Kontrastmittel unverzichtbar. Viele radiologische Untersuchungen lassen sich ohne Kontrastmittel nicht sinnvoll durchführen. Genannt seien die Urographie, die Darstellung der Blutgefäße und Herzkammern, der Liquoräume, der Lymphbahnen, des Magen-Darm-Traktes und zahlreicher pathologischer Prozesse in der Computertomographie.

Die ersten intravasal injizierbaren, relativ universell einsetzbaren jodierten Röntgenkontrastmittel wurden bereits vor mehr als 50 Jahren hergestellt. Diese Produkte, ursprünglich Salze von mono-jodierten organischen Säuren, wurden seither vielfach in ihrer Wirksamkeit und Verträglichkeit verbessert. Die besten heute verfügbaren Kontrastmittel tragen 3 oder 6 Jodatome je Molekül anstelle ursprünglich eines einzigen Jodatoms, sie sind keine Salze mehr sondern neutrale, zuckerähnliche Substanzen und die Osmolalität ihrer Lösungen ist im günstigsten Falle nicht mehr zehnfach oder mehr höher als die Osmolalität des Blutes sondern trotz extrem hoher Konzentrationen blutisoton.

Die Verträglichkeit der neuen Kontrastmittel schien zunächst alle Wünsche gleichzeitig zu erfüllen. So wurden die gleichen Produkte für sehr unterschiedliche Anwendungen wie die intravenöse Injektion, die Arteriographie, die Venographie, die Kardiographie, die Myelographie, die Darstellung des Magen-Darm-Kanals und vieler anderer Körperhöhlen verwandt. Nur die Kontrastmittelkonzentration in der Lösung und die injizierten oder infundierten Volumina wurden der speziellen Untersuchung angepaßt. Ein bekanntes Produkt erhielt den für dieses Konzept bezeichnenden Namen Omnipaque®, d. h. es sollte für alle Indikationen geeignet sein.

Dementgegen stand insbesondere in den letzten 10 Jahren eine Weiterentwicklung der einzelnen kontrastmittelverstärkten Röntgenverfahren mit zunehmend spezifischeren Anforderungen an die jeweils verwendeten Kontrastmittel. Die Gefäßdarstellung wird nicht nur zu diagnostischen Zwecken durchgeführt sondern gleichzeitig immer häufiger als wenig invasiver Zugang zu therapeutischen Eingriffen an andernfalls nur chirurgisch angehbaren pathologischen Veränderungen im Körper genutzt. Beispielsweise können im Zuge angiographischer Untersuchungen mit Darstellung der Blutgefäße über Katheter, durch die Kontrastmittel injiziert werden, gleichzeitig und mit den gleichen Kathetern Verengungen der Koronararterien aufgelöst werden, Tumoren versorgende Blutgefäße aufgeweitet werden, ohne daß der Thorax operativ eröffnet werden muß. Thromben können durch gezielte selektive Infusion von Thrombolytika in die betroffenen Blutgefäße aufgelöst werden, Tumoren versorgende Blutgefäße durch wiederum selektive Injektion von Mikropartikeln oder bestimmten, im Blut sich verfestigenden Embolisaten verschlossen werden oder mit dem letztgenannten Verfahren auch innere Blutungen gestillt oder Gefäßanomalien im Gehirn behandelt werden. Dabei werden Kontrastmittel zunächst für diagnostische Zwecke und dann vielfach wiederholt zur Kontrolle des Therapievorganges in bestimmte Gefäßregionen injiziert (interventionelle Radiologie). Da diese Vorgänge meist bei älteren, schwerkranken Patienten durchgeführt werden, sind an die Kontrastmittel extreme Anforderungen hinsichtlich der Gefäßverträglichkeit und - wegen der sich aufsummierenden Gesamtdosis - auch an die Allgemeinverträglichkeit zu stellen. Dafür sind andere Anforderungen weniger kritisch: Die modernen Bildverarbeitungstechniken erlauben es meist, mit niedrigeren Kontrastmittelkonzentrationen zu arbeiten, so daß die gewünschte niedrige Viskosität der Lösungen jetzt leichter zu erzielen ist. Weiterhin ist bekannt, daß Übelkeit und Erbrechen sowie allergieartige Reaktionen nach arterieller Kontrastmittelgabe durch Katheter seltener auftreten, als nach intravenöser Injektion mittels einer Nadel. Die Kontrastmittel für die Gefäßdiagnostik mittels Katheter sind daher auch in dieser Hinsicht nicht so kritisch zu bewerten wie intravenös injizierbare Produkte.

Als optimal für die meisten arteriellen und direkt venösen Gefäßdarstellungen sind derzeit sicherlich die sogenannten nichtionischen dimeren (hexajodierten) Kontrastmittel zu bewerten, die vor allem durch eine ausgezeichnete Gefäßverträglichkeit herausragen.

Ähnlich spezielle Anforderungen gibt es zum Beispiel an Kontrastmittel für die Darstellung anderer Körperregionen. Zur Darstellung des Spinalkanals (Myelographie) ist eine sehr gute neurale Verträglichkeit unabdingbar. Das Kontrastmittel sollte weiterhin eine ausreichend hohe Viskosität aufweisen damit die Verdünnung durch Körperflüssigkeiten möglichst langsam erfolgt. Dieselbe Anforderung der langsameren Verdünnung stellt sich bei der Darstellung der Gelenkhöhlen. Bei der Röntgendiagnostik des Magen-Darm-Traktes ist Voraussetzung, daß das Kontrastmittel einen akzeptablen Geschmack aufweist, wenig durch Osmose verdünnt wird und keine Diarrhöe auslöst. Die genannten Verfahren haben allerdings in den vergangenen Jahren eher an Bedeutung verloren, da es inzwischen andere diagnostische Methoden gibt, mit denen in vielen Fällen ein befriedigendes Ergebnis erzielt wird.

Von außerordentlicher und aufgrund neuerer technischer Entwicklungen noch weiter wachsender Bedeutung ist die Computertomographie. Anders als bei den übrigen Röntgentechniken wird nicht eine Fläche (zum Beispiel der Thorax) durchstrahlt, sondern nur eine wenige Millimeter starke Scheibe zum Beispiel durch den Schädel oder Körper und diese Scheibe als Schnittbild errechnet und dargestellt. Während die Computertomographie ursprünglich zirka 20 Sekunden benötigte, um die Daten für eine solche Scheibe zu gewinnen, ist dieser Vorgang bei modernen Geräten in 1 sec. oder 50 msec. abgeschlossen. Gleichzeitig kann der Patient durch die Meßeinrichtung bewegt werden, so daß beispielsweise in 30 sek. 30 Schichten zu 5 mm Schichtdicke erfaßt werden und damit ein Körperabschnitt von 15 cm Länge.

Die gebräuchlichen Kontrastmittel lassen nach intravenöser Injektion die Blutgefäße erkennen, zeigen die Perfusion von Gefäßen, Organen und Geweben an, sowie die Permeabilität von Kapillaren durch Übertritt aus dem Blutraum in den deutlich größeren Zwischenzellraum der Gewebe.

Viele pathologische Veränderungen lassen sich besonders gut während der ersten Passage des Kontrastmittels nach schneller intravenöser Injektion erkennen, da dann die Verteilungsunterschiede zu dem gesunden Gewebe am ausgeprägtesten sind. Es ist klar, daß schnelle Computertomographen, die binnen weniger Sekunden ganze Körperabschnitte erfassen oder durch rasch wiederholte Aufnahmen den zeitlichen Ablauf der Kontrastmittelpassage durch eine bestimmte Region darstellen, diese Kontrastmittelverteilungsunterschiede am besten erkennen können. Die schnelle Computertomographie ist daher zu einem zunehmend bedeutenderen und immer häufiger genutzten Mittel der medizinischen Diagnostik geworden. Etwa die Hälfte der derzeit gebrauchten Röntgenkontrastmittelmengen wird in der Computertomographie eingesetzt.

Die Anwendungsweise und die Anforderungen an die Kontrastmittel in der Computertomographie unterscheiden sich deutlich von den Anforderungen für die Katheterarteriographie und Venographie. Um ausreichende Kontraste zu erzielen, müssen hohe Mengen der Kontrastmittel rasch durch verhältnismäßig enge Kanülen intravenös injiziert werden. Erforderlich sind Kontrastmittelmengen entsprechend 15-45 g Jod je Einzelinjektion. Es kann notwendig werden - oder ist sogar gebräuchlich - die Einzelinjektionen im Abstand von wenigen Minuten zu wiederholen. Die Injektionsgeschwindigkeit beträgt bei den leistungsfähigen schnellen Computertomographen im allgemeinen 1-5 ml/sec. oder mehr (Small W. C., Nelson R. C., Bernadino M. E., Brummer L. T.: Contrast enhanced spiral CT of the liver: Effects of different amounts and injection rates of contrast material on early contrast enhancement. AJR 163, 87-92, 1994).

Um diese Mengen so schnell injizieren zu können, müssen die Kontrastmittellösungen bei hoher Jodkonzentration ausreichend dünnflüssig sein. Die nach intravenöser Gabe häufigsten und störendsten Kontrastmittelnebenwirkungen (Dawson P., Clauß W. Edts: Contrast Media in Practice, Springer Verlag Berlin Heidelberg 1993, S. 107-109) wie Übelkeit, Erbrechen und allergieartige Reaktionen sollten möglichst selten auftreten. Extrem hohe Dosierungen müssen von allen Organen trotz entsprechender Vorschädigung bei vielen Patienten vertragen werden.

Die Kontrastmittel sollten zu vertretbaren Kosten herstellbar sein, um die an sich noch relativ kostengünstige computertomographische Untersuchungsmethode in ihrer Verfügbarkeit nicht unnötig einzuschränken. Da die intravenöse Injektion nur selten zu Reizungen der lokalen Venen oder nach Verdünnung im Herzen gar der Arterien führt, werden an die lokale Gefäßverträglichkeit der Kontrastmittel für die Computertomographie keine extremen Anforderungen gestellt. Ein Kontakt mit dem Zentralnervensystem ist nur nach mindestens 10 facher Verdünnung durch den allgemeinen Blutkreislauf möglich, so daß die neurale Verträglichkeit fast aller intravasalen Kontrastmittel voll befriedigend sein sollte.

Ähnliche Anforderungen wie in der Computertomographie sind auch an Kontrastmittel für die Ausscheidungsurographie zu stellen. Auch diese Kontrastmittel werden rasch intravenös verabreicht. Die spezifischen Erkrankungen der für die Urographie vorgesehenen Patienten sowie die Notwendigkeit einer kontrastreichen und vollständigen röntgenologischen Darstellung der Harnwege stellen zusätzliche Anforderungen an die Verträglichkeit, Ausscheidung und die diuretischen Effekte.

Viele der heute verfügbaren wasserlöslichen Röntgenkontrastmittel und der Neuentwicklungen der letzten Jahre versuchen den Anforderungen der unterschiedlichsten Anwendungsrichtungen gleichzeitig gerecht zu werden, andere sind speziell auf die angiographische Anwendung zugeschnitten. Einige weisen die unterschiedlichsten Mängel auf und sind für keine der genannten Anwendungen optimal geeignet. Die Substanzen (lopentol, lohexol, loversol) enthalten beispielsweise einen relativ geringen Jodgehalt, was zu einer erhöhten Viskosität ihrer Lösungen führt. Gleichzeitig steigt die Osmolalität unerwünscht an. Nichtionische dimere Kontrastmittel (lotrolan, lodixanol) sind erwünschterweise blutisoton, jedoch gleichzeitig noch viskoser. Neue Kontrastmittel mit sehr hohem Jodgehalt (beispielsweise, die in den Patentschriften US 5047228 und US 5019371 genannten Verbindungen) schienen niedrige Osmolalität und Viskosität auf ideale Weise miteinander zu verbinden. Ungenügende Wasserlöslichkeit und Verträglichkeitsprobleme haben jedoch bisher eine erfolgreiche Entwicklung verhindert.

Die bisher den Anforderungen der Computertomographie am besten angepaßte Gruppe von Kontrastmitteln besteht aus chemisch sehr engverwandten Verbindungen: lopromid, lopamidol und lomeprol. Sie zeichnen sich durch niedrige Viskosität (für rasche Injektion) bei niedriger Osmolalität (für geringe Herz-Kreislauf Belastung) und akzeptable Allgemein- und Organverträglichkeit aus. Damit kommen sie den Anforderungen an schnell und in hoher Dosis intravenös injizierbare Kontrastmittel am nächsten.

Die stete Entwicklung der Computertomographie und die wiederholte Untersuchung schwerstkranker Patienten erfordert jedoch eine weitere Verbesserung insbesondere der Verträglichkeit der für diese Anwendung vorgesehenen Röntgenkontrastmittel, ohne daß wie bei den übrigen genannten Substanzen der Jodgehalt vermindert und damit die Viskosität erhöht und die Injizierbarkeit verschlechtert wird.

Der Erfindung liegt somit die Aufgabe zugrunde, derartige Verbindungen und Mittel zur Verfügung zu stellen, sowie ein Verfahren zu ihrer Herstellung zu schaffen. Die Lösung dieser Aufgabe erfolgt durch den in den Patentansprüchen gekennzeichneten Gegenstände.

Es wurde gefunden, daß zwei neue Verbindungen überraschend günstige Eigenschaften zeigen, insbesondere bei der Anwendung in der Computertomographie. Es handelt sich dabei um Verbindungen der allgemeinen Formel I 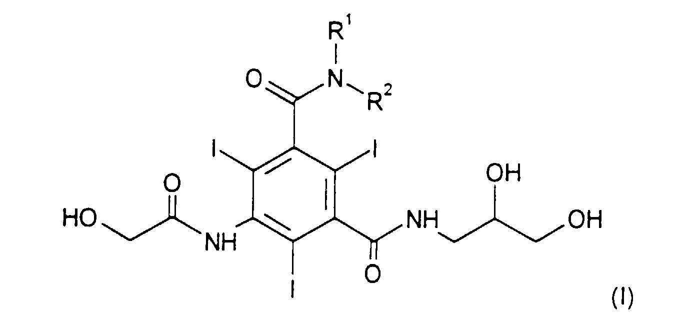 worin entweder
- R¹ für den Methylrest und R² für den 2,3-Dihydroxypropylrest steht oder
- R¹ und R² jeweils für den 2-Hydroxyethylrest stehen.

Während Osmolalität, Viskosität und Jodgehalt den strukturisomeren Verbindungen lopamidol und lomeprol sehr ähnlich sind, zeigt sich eine überraschend erhöhte Hydrophilie der erfindungsgemäßen Verbindungen (Tab. 1).

**Tab.1:**

| Butanol/Wasser (Puffer pH 7) - Verteilungskoeffizient unterschiedlicher Kontrastmittel, n = 4, Mittelwert ± Standardabweichung und Jodgehalt des Moleküls | | |
|---|---|---|
| | Verteilungskoeffizient | Jodgehalt (%) |
| Beispiel 1 | 0.069 ± 0.011 | 49 |
| lopromid | 0.149 ± 0.011 | 48 |
| lopamidol | 0.089 ± 0.014 | 49 |
| lomeprol | 0.105 ± 0.006 | 49 |
| lohexol | 0.082 ± 0.005 | 46 |

Die Substanz nach Beispiel 1 weist den geringsten Verteilungskoeffizienten der strukturell vergleichbaren Verbindungen auf, d. h. sie ist am stärksten hydrophil. Die Hydrophilie wird nicht wie bei der mit lohexol bezeichneten Verbindung durch die Einführung einer zusätzlichen Hydroxyalkylfunktion erreicht (die den Jodgehalt vermindert und Viskosität und Osmolalität erhöht), sondern ist die überraschende Folge einer neuen sterischen Anordnung der Substituenten an dem jodierten Aromaten.

Die Hydrophilie wird als eine wichtige Voraussetzung für eine Verminderung der bei intravenöser Injektion beobachteten nicht osmolalitätsbedingten Nebenwirkungen angesehen (Dawson P., Clauß W.: Contrast Media in Practice, Springer Verlag Berlin Heidelberg 1993, Seite 11-12). Dazu gehören vor allem Übelkeit und Erbrechen, sowie allergieartige Reaktionen bis hin zum schweren anaphylaktoiden Schock. Andere Testergebnisse, die eine besonders gute Allgemeinverträglichkeit anzeigen, werden in biochemischen, biologischen und toxikologischen (Tab. 2) Untersuchungen gewonnen.

**Tab.2:**

| Untersuchungen der letalen Dosis nach intravenöser Injektion bei Ratten (90-110 g), Konzentration der Kontrastmittellösungen entsprechend 300 mg Jod/ml, Injektionsgeschwindigkeit 2 ml/min, Dosis entsprechend g Jod/kg Körpergewicht; Angabe der Zahl der gestorbenen Tiere / Gesamtzahl der Tiere | | | | |
|---|---|---|---|---|
| Prüfsubstanz | Dosis 1: 12 g J/kg | Dosis 2: 15 g J/kg | Dosis 3: 18 g J/kg | alle Dosierungen (%) |
| Beispiel 1 | 0/4 | 2/4 | 2/4 | 33 |
| lopamidol | 2/4 | 1/4 | 4/4 | 58 |
| lopromid | 1/4 | 3/4 | 4/4 | 67 |
| lomeprol | 2/3 | 3/3 | 3/3 | 89 |

Prüfungen der Organtoxizität ergeben eine bessere Verträglichkeit der erfindungsgemäßen Verbindungen, wie sie in der Computertomographie von zunehmender Bedeutung ist. Die Ausscheidung erfolgt besonders rasch und vollständig. Schließlich weisen die erfindungsgemäße Verbindungen eine hohe Stabilität bei unterschiedlichen Lagerungsbedingungen auf. Dadurch ist eine hervorragende Reinheit auch unter den Bedingungen der Praxis und zum Zeitpunkt der Anwendung gewährleistet.

Die Erfindung betrifft daher die in den Patentansprüchen gekennzeichneten neuen Verbindungen.

Die Computertomographie mit schnellen Geräten ist ein bildgebendes Diagnoseverfahren, das außerordentlich detaillierte und genaue Informationen in kürzester Zeit generiert. Bei hohem Patientendurchsatz pro Zeit ist es trotz aufwendiger Technik ein kostengünstiges Verfahren. Die erfindungsgemäßen Kontrastmittel tragen wesentlich zu einer Verbesserung des Verfahrens bei, indem sie trotz rascher Injektion und hoher Dosierung einen durch Nebenwirkungen weitgehend ungestörten Betrieb erlauben. Gleichzeitig tragen die Eigenschaften der Kontrastmittel auch zur Schonung der häufig schwerstkranken Patienten bei.

Die Erfindung betrifft daher auch die Verwendung der in den Patentansprüchen gekennzeichneten Verbindungen zur Herstellung von Mittel für die Diagnostik mittels Computertomographie.

Durch die gute renale Ausscheidung der erfindungsgemäßen Verbindungen in Kombination mit der guten Verträglichkeit auch bei schwerkranken Patienten sind die Verbindungen hervorragend für die Urographie geeignet.

Die erfindungsgemäßen Verbindungen sind wegen ihrer guten Verträglichkeit ebenfalls für Angiegraphie, die Myelographie und die interventionelle Radiologie geeignet. Die Erfindung betrifft daher weiterhin die Verwendung der in den Patentansprüchen gekennzeichneten Verbindungen zur Herstellung von Mitteln für die Urographie, für die Angiographie, für die Myelographie und für die interventionelle Radiologie.

Zum Zwecke der Anwendung wird die Kontrastmittelsubstanz in unterschiedlicher Konzentration in sterilem, pyrogenfreiem Wasser gelöst. Die Konzentrationen entsprechen ca. 20 mg bis 1 g Kontrastmittelsubstanz/ml, entsprechend ca. 10 bis 500 mg Jod/ml. Bevorzugt sind für die besonders vorteilhaften parenteralen Anwendungen Konzentrationen entsprechend 100-400 mg Jod/ml. Den Kontrastmittellösungen können in üblicher Weise physiologisch verträgliche Puffer wie Natriumcarbonat, Tris(Trishydroxymethylaminomethan)/HCI, Bicarbonat, Phosphat, Citrat, etc. zugesetzt werden. Die Pufferkonzentration kann 1-100 mmol/Liter betragen. Der Puffer wird bevorzugt auf annähernd physiologische pH-Werte zwischen 5 und 8 eingestellt. Den Kontrastmittellösungen können auch Komplexbildner, wie EDTA, DTPA und/oder deren Salze mit physiologisch verträglichen Ionen wie Natrium, Kalium, Magnesium, Calcium, Lysin etc. zugesetzt werden, sowie pharmakologisch aktive Substanzen (Gefäßdilatatoren, Gerinnungshemmer, etc.), die die Verträglichkeit verbessern oder die Pharmakokinetik in erwünschter Weise verändern.

Eine weitere Anwendung der betreffenden Kontrastmittel kann die orale Einnahme zur Darstellung des Magen-Darm-Traktes sein. Für diesen Zweck kann das Kontrastmittel als Pulver zur Herstellung einer Lösung vor Gebrauch oder als Konzentrat oder als fertige Lösung angeboten werden. In jedem Falle kann das Kontrastmittel physiologisch verträgliche Puffer, Stabilisatoren, Substanzen zur Anpassung der Osmolalität, pharmakologisch wirksame Substanzen, Konservierungsmittel, Geschmacksstoffe und/oder Quellstoffe enthalten. Im allgemeinen werden die erfindungsgemäßen Mittel in Mengen von 2 bis 1500, vorzugsweise 20 bis 1000 ml/Untersuchung, dosiert.

Die Abfüllung der Kontrastmittellösungen erfolgt in Glasbehälter oder inerte Kunststoffbehältnisse in den für die Röntgenuntersuchungen üblichen Volumina von wenigen Millilitern pro Einheit bis zu ca. 1 Liter. Die Lösungen können entweder steril filtriert und unter sterilen Bedingungen in sterile Behältnisse abgefüllt und diese steril verschlossen werden oder die Lösungen werden in den Behältnissen hitzesterilisiert.

Die Dosierung je Patient beträgt ebenfalls wenige Milliliter bis maximal ca. 1 Liter, wobei die Kontrastmitteldosis etwa 1-150 g Jod pro Patient entspricht, bevorzugt sind 20-100 g Jod.

Die Erfindung betrifft daher auch die in den Patentansprüchen gekennzeichneten pharmazeutischen Mittel.

Die Erfindung betrifft ferner ein Verfahren zur Herstellung von Verbindungen allgemeinen Formel I, dadurch gekennzeichnet, daß eine Verbindung der allgemeinen Formel II 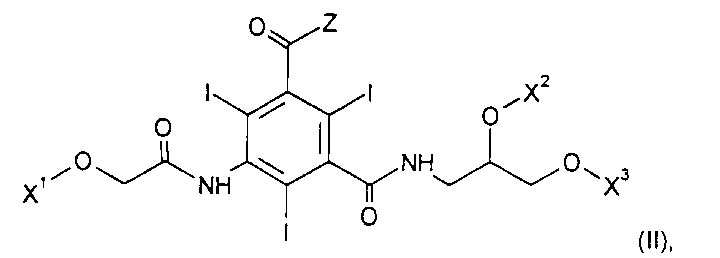 worin
X¹, X² und X³ für Hydroxyschutzgruppen stehen und Z für einen reaktiven Säure- oder Esterrest steht, in einem polaren Lösungsmittel oder einem Lösungsmittelgemisch, enthaltend mindestens ein polares Lösungsmittel, bei einer Temperatur von 0°C bis 120°C, gegebenenfalls in Anwesenheit einer Hilfsbase mit Diethanolamin oder N-Methylamino-propandiol umgesetzt wird und anschließend die Hydroxyschutzgruppen in bekannter Weise abgespalten werden.

Als reaktiver Säure- oder Esterrest Z kommen insbesondere Halogenatome, wie Chlor-, Brom- oder lodatome in Betracht. Durchführbar ist das Verfahren aber auch, wenn Z für den Azidrest, den Alkoxycarbonyloxyrest oden den Rest einer reaktiven Estergruppe (z.B. einen Alkyl-O-, Aryl-O- oder N=-C-CH₂- Rest) steht. Bevorzugte Reste Z sind Halogenatome, besonders bevorzugt ist das Chloratom.

Als Hydroxyschutzgruppen kommen diejenigen Gruppen in Betracht, die bekanntermaßen für einen intermediären Hydroxygruppenschutz geeignet sind, d.h. die sich leicht einführen und sich später unter Rückbildung der letztlich gewünschten freien Hydroxygruppe auch wieder leicht abspalten lassen. Bevorzugt ist der Schutz durch Veresterung, z.B. durch Einführung des Benzoyl-, Alkanoyl- oder Acyl- insbesondere des Acetyl- oder des Acetoxyacetylrestes. Die vicinalen Hydroxygruppen können auch gemeinsam durch Einführung des cyclischen Sulfitesters oder des Kohlensäureesters geschützt werden. Geeignete Schutzgruppen sind auch Ethergruppen wie z.B. Benzyl-, Di- und Triphenylmethyl-ethergruppen sowie Acetal- und Ketalgruppen mit z.B. Acetaldehyd oder Aceton.

Die Verbindungen der allgemeinen Formel II mit den genannten Aminen wird in wenig polaren bis polaren Lösungsmitteln gegebenenfalls in Gegenwart von Hilfsbasen durchgeführt. Steht Z für ein Halogenatom wird die Reaktion stets in Anwesenheit einer Hilfsbase durchgeführt. Als Hilfsbasen werden tertiäre Amine, z.B. Trialkylamine oder Pyridin, oder anorganische Basen wie Alkali- oder Erdalkalihydroxide, -carbonate, oder -hydrogencarbonate, z.B. Natriumhydroxid, Natriumhydrogencarbonat, Kaliumcarbonat, Natriumcarbonat, Calciumhydroxid oder Magnesiumhydroxid. Vorzugsweise werden Natriumcarbonat, Kaliumcarbonat, Triethylamin und Tributylamin verwendet. Die Hilfsbase, die den bei der Reaktion entstehenden Halogenwasserstoff bindet wird so gewählt, daß sich das Salz der Hilfsbase in dem entsprechend gewählten Lösungsmittel möglichst quantitativ kristallin abscheidet und durch einfache Filtration abgetrennt werden kann. Die Temperatur bei der Reaktion kann zwischen 0°C und 120°C liegen, vorzugsweise zwischen 30°C und 90°C. Bevorzugte Lösungsmittel sind Aceton, Dioxan, Dimethoxyethan, Diethylenglykoldimethylether, Dimethylacetamid und Dimethylformamid und deren Gemische sowie deren Gemische mit Wasser.

Die Abspaltung der Hydroxyschutzgruppen erfolgt nach Methoden, die dem Fachmann geläufig sind. Sie kann ohne Isolierung des Zwischenproduktes mit der Aufarbeitung und Isolierung der Umsetzungsprodukte erfolgen. Sie kann aber auch in einer getrennten Reaktionsstufe durchgeführt werden. Acylschutzgruppen können beispielsweise durch alkalische und Acetal-, Ketal- oder Etherschutzgruppen durch saure Hydrolyse abgespalten werden. Die alkalische oder saure Hydrolyse, insbesondere die alkalische Hydrolyse, wird bevorzugt in Wasser durchgeführt. Als Base dienen Alkalihydroxide, bevorzugt Natriumhydroxid.

Die anorganischen und/oder organischen Salze, die im Reaktionsverlauf entstehen werden durch Behandlung der Reaktionslösungen mit lonenaustauschern oder Adsorbenzien (z.B.: Diaion oder Amberlite XAD-2 oder -4) abgetrennt und die salzfreien Produkte durch Kristalliation aus organischen Solventien, insbesondere Ethanol, weiter gereinigt.

Die Verbindungen der allgemeinen Formel II werden ausgehend von den in der EP 308364 beschriebenen Verbindungen 5-Amino-2,4,6-triiod-isophthalsäure-(2,3-dihydroxy-propyl)-monoamid oder 5-Amino-2,4,6-triiod-isophthalsäure-(2,3-diacetoxy-propyl)-monoamid auf dem Fachmann bekannte Weise (z.B.: Methoden der organischen Chemie (Houben-Weyl), Bd. E5, Carbonsäuren und Carbonsäure-Derivate, Thieme Verlag, Stuttgart, New York, 1985) hergestellt.

Zur Herstellung der besonders bevorzugten Verbindungen mit Z in der Bedeutung eines Chloratoms wird die Umsetzung beispielsweise in unpolaren bis polaren, vorzugsweise mäßig polaren, aprotischen Lösungsmitteln mit organischen und/oder anorganischen Säurehalogeniden, vorzugsweise Säurechloriden durchgeführt. Die Reaktion wird bei Temperaturen zwischen 0°C und 120°C, vorzugsweise bei 50° bis 90°C durchgeführt. Die Reaktion kann in An- oder Abwesenheit von basischen Katalysatoren wie Pyridin oder 4-Dimethylamino-pyridin erfolgen.
Vorzugsweise wird die Umsetzung in Gegenwart eines basischen Katalysators in einem wenig polaren aprotischen Lösungsmittel durchgeführt, aus dem das Säurechlorid der allgemeinen Formel I sich kristallin abscheidet und leicht und in guter Ausbeute und Reinheit isoliert werden kann. Bevorzugte Lösungsmittel sind Essigester, Isopropylacetat, Dioxan, Dimethoxyethan, Diethylenglykoldimethylether, Dimethylacetamid und Dimethylformamid.

Wird 5-Amino-2,4,6-triiod-isophthalsäure-(2,3-dihydroxy-propyl)-monoamid als Ausgangsmaterial verwendet, so kann wahlweise zuerst mit Acetoxyacetylchlorid und anschließend mit z. B. dem anorganischen Säurehalogenid Thionylchlorid zu dem Intermediat 5-Acetoxyacetylamino-2,4,6-triiod-isophthalsäure-mono-(2,3-diacetoxyacetoxy-propyl)-amid-chlorid oder zuerst mit Thionylchlorid und anschließend mit Acetoxyacetylchlorid zu dem Intermediat 5-Acetoxyacetylamino-2,4,6-triiod-isophthalsäure-mono-(2-oxo-1,3,2-dioxathiolan-4-ylmethyl)-amid-chlorid umgesetzt werden.

Die folgenden Beispiele sollen den Erfindungsgegenstand erläutern, ohne ihn auf diese beschränken zu wollen.

### Beispiel 1: 5-Hydroxyacetylamino-2,4,6-triiod-isophthalsäure-[(2,3-dihydroxy-N-methyl-propyl)-(2,3-dihydroxypropyl)]-diamid

1.1) Herstellung der Säurechloride
   1.1.1) 5-Acetoxyacetylamino-2,4,6-triiod-isophthalsäure-mono-(2,3-diacetoxypropyl)amid-chlorid
      200 g 5-Amino-2,4,6-triiod-isophthalsäure-(2,3-diacetoxy-propyl)-monoamid werden in 200 ml Dioxan suspendiert, 114.4 g Acetoxyacetylchlorid hinzugefügt und die Suspension auf 90°C erwärmt. Es entsteht eine Lösung. Nach ca. 2 Stunden zeigt die DC in z. B. Methylenchlorid/Methanol 10:3, daß die Aminoguppe der Ausgangsverbindung quantitativ acyliert ist. Es werden 49.84g Thionylchlorid zugefügt und weitere 2 Stunden bei 90°C gerührt. Die DC im vorgenannten System zeigt dann die quantitative Umsetzung zum Säurechlorid an. Die Reaktionslösung wird durch Destillation im Vakuum auf ca. ein Drittel ihres Volumens verringert, 350 ml Essigester zugefügt und 2 Stunden bei Raumtemperatur gerührt. Es entsteht reichlich Kristallisat. Dieses wird abfiltriert, mit Essigester gewaschen und bei 50 °C im Vakuum getrocknet. Die Ausbeute beträgt 212g = 91 % d. Th.
   1.1.2) 5-Acetoxyacetylamino-2,4,6-triiod-isophthalsäure-mono-(2,3-diacetoxypropyl)amid-chlorid
      71.6 g 5-Amino-2,4,6-triiod-isophthalsäure-(2,3-diacetoxy-propyl)-monoamid werden in 71.6 ml Essigester suspendiert, 34.71 g Acetoxyacetylchlorid hinzugefügt, und die Suspension 4 Stunden bei einer Innentemperatur von ca. 82 °C gerührt. Dann werden 23,8 g Thionylchlorid zugefügt und weitere 5 Stunden bei ca. 85°C Innentemperatur gerührt. Die Suspension wird dann mit 200 ml Essigester verdünnt, auf Raumtemperatur gekühlt, das Kristallisat abfiltriert, mit Essigester gewaschen und im Vakuum bei 50°C getrocknet. Ausbeute 75.5 g = 90.5% d. Th..
   1.1.3) 5-Acetoxyacetylamino-2,4,6-triiod-isophthalsäure-mono-(2,3-diycetoxypropyl)amid-chlorid
      71.6 g 5-Amino-2,4,6-triiod-isophthalsäure-(2,3-diacetoxy-propyl)-monoamid werden in 71.6 ml Essigester suspendiert, 34.71 g Acetoxyacetylchlorid und 0.54 g 4-Dimethylamino-pyridin (DMAP) hinzugefügt und die Suspension 2 Stunden bei einer Innentemperatur von ca. 82 °C gerührt. Dann werden 23,8 g Thionylchlorid zugefügt und weitere 5 Stunden bei ca. 85°C Innentemperatur gerührt. Die Suspension wird dann mit 200 ml Essigester verdünnt, auf Raumtemperatur gekühlt das Kristallisat abfiltriert, mit Essigester gewaschen und im Vakuum bei 50°C getrockent. Ausbeute 71.8 g = 86% d. Th..
   1.1.4) 5-Acetoxyacetylamino-2,4,6-triiod-isophthalsäure-mono-(2,3-diacetoxypropyl)amid-chlorid
      71.6 g 5-Amino-2,4,6-triiod-isophthalsäure-(2,3-diacetoxy-propyl)-monoamid werden in 71.6 ml Diethylenglykoldimethylether suspendiert, 27.31 g Acetoxyacetylchlorid hinzugefügt und die Suspension 8 Stunden bei einer Innentemperatur von ca. 95 °C gerührt. Es entsteht eine Lösung. Dann werden 29.74 g Thionylchlorid zugefügt und weitere 2.5 Stunden bei ca. 83°C Innentemperatur gerührt. Die Lösung wird danach mit 200 ml Essigester verdünnt, auf Raumtemperatur gekühlt das Kristallisat abfiltriert, mit Essigester gewaschen und im Vakuum bei 50°C getrockent. Ausbeute 67.2 g = 80.5% d. Th..
   1.1.5) 5-Acetoxyacetylamino-2,4,6-triiod-isophthalsäure-mono-(2,3-diacetoxypropyl)amid-chlorid
      357 g 5-Amino-2,4,6-triiod-isophthalsäure-(2,3-diacetoxy-propyl)-monoamid werden in 1.6 1 Dichlorethan mit 178 ml Thionylchlorid 1.5 Stunden am Rückfluß gekocht. Aus der Suspension entsteht eine Lösung. Diese wird auf Raumtemperatur gekühlt und mit gesättigter Natriumhydrogencarbonat-Lösung ausgerührt, bis die wäßrige Phase schwach alkalisch reagiert. Die Phasen werden getrennt, die organische Phase zum Öl eingedampft, das Öl in 1 l Essigester gelöst, mit 204 g Acetoxyacetylchlorid versetzt und ca. 8 Stunden am Rückfluß gekocht. Aus der Lösung scheidet sich zunehmend 5-Acetoxyacetylamino-2,4,6-triiod-isophthalsäure-mono-(2,3-diacetoxypropyl)-amid-chlorid kristallin ab. Es wird auf Raumtemperatur gekühlt, abgesaugt, mit Essigester gewaschen und im Vakuum bei 50°C getrocknet. Die Ausbeute beträgt 308 g = 72.3 % d. Th.
   1.1.6) 5-Acetoxyacetylamino-2,4,6-triiod-isophthalsäure-(2-oxo-1,3,2-dioxathiolan-4-ylmethyl)-monoamid-chlorid
      119.08 g 5-Amino-2,4,6-triiod-isophthalsäure-(2,3-dihydroxy-propyl)-monoamid werden in 1070 ml Essigester suspendiert, 85.44 g Thionylchlorid zugefügt und die Suspension zum Sieden erhitzt. Nach ca. 20 Minuten entsteht eine klare Lösung. Zu dieser werden im Verlaufe von ca. 10 Minuten 90 g Acetoxyacetylchlorid zugefügt und weiter bei Siedetemperatur gerührt. Nach ca. 4 Stunden kristallisiert das Produkt aus der siedenden Reaktionslösung aus. Nach ca. 8 Stunden ist die Umsetzung beendet. Es wird auf Raumtemperatur gekühlt, das Kristallisat abfiltriert, mit Essigester gewaschen und bei 50°C im Vakuum getrocknet. Ausbeute 97.86 g = 67.24% d. Th.
   1.1.7) 5-Acetoxyacetylamino-2,4,6-triiod-isophthalsäure-(2-oxo-1,3,2-dioxathiolan-4-ylmethyl)-monoamid-chlorid
      63.1 g 5-Amino-2,4,6-triiod-isophthalsäure-(2,3-dihydroxy-propyl)-monoamid werden in 120 ml Essigester suspendiert, bei ca. 5-10°C 12.5 g Thionylchlorid allmählich hinzugefügt und 3 Stunden bei Raumtemperatur gerührt. Die Suspension wird dann zum Sieden erhitzt, im Verlaufe einer Stunde 41 g Acetoxyacetylchlorid eingetropft, 0. 6g 4-Dimethylamino-pyridin zugefügt und 3 Stunden am Rückfluß gekocht. Die Suspension geht in eine Lösung über. Nach Ablauf dieser Zeit werden in diese Lösung 23.8 g Thionylchlorid im Verlaufe von 30 Minuten eingetropft und danach 2 Stunden am Rückfluß gekocht. Das Produkt scheidet sich in dieser Zeit zunehmend als Kristallisat aus der Lösung ab. Es werden 200 ml Essigester zugefügt, weitere 30 Minuten am Rückfluß gekocht, dann auf Raumtemperatur gekühlt, das Kristallisat abgesaugt, mit Essigester gewaschen und bei 50°C im Vakuum 24 Stunden getrocknet. Ausbeute: 58.5 g = 73.5% d. Th..
   1.1.8) 5-Acetoxyacetylamino-2,4,6-triiod-isophthalsäure-mono-(2,3-diacetoxyacetoxy-propyl)-amid-chlorid
      63.1 g 5-Amino-2,4,6-triiod-isophthalsäure-(2,3-dihydroxy-propyl)-monoamid werden in 100 ml Essigester suspendiert, 60.4 g Acetoxyacetylchlorid und 1 g 4-Dimethylamino-pyridin zugefügt und die Suspension am Rückfluß gekocht. Die Suspension geht allmählich in eine Lösung über. Nach ca. 4 Stunden ist die Acylierung vollständig. Es werden 14.7 ml Thionylchlorid zugefügt und weitere 5 Stunden am Rückfluß gekocht. Das Produkt beginnt sich kristallin abzuscheiden. Um das Kristallisat zu vermehren werden weitere 200 ml Essigester zugefügt, auf Raumtemperatur gekühlt und 2 Stunden nachgerührt. Das Kristallisat wird abgesaugt, mit Essigester gewaschen und bei 50°C im Vakuum getrocknet. Ausbeute: 64.8 g = 68.2% d. Th.
1.2) Herstellung von 5-Hydroxyacetylamino-2,4,6-triiod-isophthalsäure-[( 2,3-dihydroxy-N-methyl-propyl)-(2,3-dihydroxypropyl)]-diamid
   1.2.1) 500 g 5-Acetoxyacetylamino-2,4,6-triiod-isophthalsäure-mono-(2,3-diacetoxypropyl)-amid-chlorid (Bsp. 1.1.1) werden in 1300 ml Aceton suspendiert, 69.2 g N-Methyl-aminopropandiol und 188.55g Na₂CO₃ x 10 H₂O zugefügt und die Suspension 1 Stunde am Rückfluß gekocht. Die anorganischen Salze werden abfiltriert und das Filtrat bei 50 °C im Verlaufe von ca. 1 Stunde mit insgesamt 100ml 50 Gew.%-iger Natronlauge versetzt. Die Lösung wird dann mit 27 ml 11.5-normaler Salzsäure auf pH 7 gebracht, an lonenaustauschern entsalzt, das wäßrige Eluat im Vakuum zu einem Öl eingedampft und dieses aus 2 I Ethanol in der Siedehitze kristallisiert.
      Ausbeute: 295.6 g = 63.5% d. Th.
   1.2.2) 90 g 5-Acetoxyacetylamino-2,4,6-triiod-isophthalsäure-mono-(2,3-diacetoxypropyl)-amid-chlorid (Bsp. 1.1.2) wurden in 260 ml Dioxan gelöst, 47.3 g Na₂CO₃ x 10 H₂O und 13.86 g N-Methyl-aminopropandiol zugefügt und 3 Stunden bei Raumtemperatur gerührt. Die anorganischen Salze wurden abgesaugt, das Filtrat im Vakuum zu einem Öl eingeengt, dieses Öl in 200 ml Wasser gelöst und bei 40°C allmählich mit 35 ml 50 Gew.%-iger Natronlauge versetzt. Die wäßrige Lösung wurde dann mit ca. 6 ml 11.5-normaler Salzsäure auf pH 7 gebracht, an lonenaustauschern entsalzt, das wäßrige Eluat im Vakuum zu einem Öl eingedampft und dieses aus 350 ml Ethanol in der Siedehitze kristallisiert. Ausbeute: 58.1 g = 68% d. Th.
   1.2.3) 100 g 5-Acetoxyacetylamino-2,4,6-triiod-isophthalsäure-(2-oxo-1,3,2-dioxathiolan-4-ylmethyl)-monoamid-chlorid (Bsp. 1.1.7) werden in 500 ml Aceton suspendiert, 39.5 g Sodadekahydrat und 14.5 g Methylaminopropandiol zugefügt und 1 Stunde am Rückfluß gekocht. Es wird dann auf Raumtemperatur gekühlt, die anorganischen Salze abfiltriert, das Filtrat unter vermindertem Druck weitgehend eingedampft und der Rückstand in 300 ml Wasser gelöst. Bei 40°C wird die wäßrige Lösung allmählich mit ca. 20 ml 50Gew.%-iger NaOH versetzt und dabei der pH-Wert zwischen 10 und 12 gehalten. Die basiche Lösung wird anschließend an einem Kationen- und einem Anionenaustauscher entsalzt, das wäßrige Eluat zu einem Öl eingedampft und dieses aus ca. 300 ml Ethanol kristallisiert. Ausbeute: 61.5 g = 63% d. Th.

### Beispiel 2: 5-Hydroxyacetylamino-2,4,6-triiod-isophthalsäure-(2,3-dihydroxypropyl)-[N-bis(2-hydroxyethyl)]-diamid

2.1) 100 g 5-Acetoxyacetylamino-2,4,6-triiod-isophthalsäure-(2-oxo-1,3,2-dioxathiolan-4-ylmethyl)-monoamid-chlorid (Bsp. 1.1.7) werden in 500 ml Aceton suspendiert, 39.5 g Sodadekahydrat und 14.5 g Diethanolamin zugefügt und 1 Stunde am Rückfluß gekocht. Es wird dann auf Raumtemperatur gekühlt, die anorganischen Salze abfiltriert, das Filtrat unter vermindertem Druck weitgehend eingedampft und der Rückstand in 300 ml Wasser gelöst. Bei 40°C wird die wäßrige Lösung allmählich mit ca. 20 ml 50 Gew.%-iger NaOH versetzt und dabei der pH-Wert zwischen 10 und 12 gehalten. Die basische Lösung wird anschließend an einem Kationen- und einem Anionenaustauscher entsalzt, das wäßrige Eluat zu einem Öl eingedampft und dieses aus ca. 300 ml Ethanol kristallisiert. Ausbeute: 68.6 g = 70.3% d. Th.
2.2) 95 g 5-Acetoxyacetylamino-2,4,6-triiod-isophthalsäure-mono-(2,3-diacetoxyacetoxy-propyl)-amid-chlorid (Bsp. 1.1.8) werden in 500 ml Aceton suspendiert, 31.5 g Sodadekahydrat und 11.6 g Diethanolamin zugefügt und 1 Stunde am Rückfluß gekocht. Es wird dann auf Raumtemperatur gekühlt, die anorganischen Salze abfiltriert, das Filtrat unter vermindertem Druck weitgehend eingedampft und der Rückstand in 300 ml Wasser gelöst. Bei 40°C wird die wäßrige Lösung allmählich mit ca. 16 ml 50Gew.%-iger NaOH versetzt und dabei der pH-Wert zwischen 10 und 12 gehalten. Die basiche Lösung wird anschließend an einem Kationen- und einem Anionenaustauscher entsalzt, das wäßrige Eluat zu einem Öl eingedampft und dieses aus ca. 300 ml Ethanol kristallisiert. Ausbeute: 51 g = 65.7% d. Th.

### Beispiel 3: Lösung zur Injektion für die Computertomographie

683,7 g der wasser- und pyrogenfreien Substanz nach Beispiel 1 werden zu 500 ml pyrogenfreiem Wasser enthaltend 10 mmol Tris (Trishydroxymethylaminomethan) und 100 mg Na₂Ca EDTA gegeben, in der Wärme gelöst, abgekühlt, mit 1 n HCI auf pH 6,8 eingestellt und mit pyrogenfreiem Wasser auf 1000 ml aufgefüllt. Die Lösung wird zu Portionen von 200 ml in 250 ml fassende Glasflaschen abgefüllt, die mit Gummistopfen dicht verschlossen, verbördelt und für 20 min bei 121 °C hitzesterilisiert werden.

### Beispiel 4: Anwendung in der Computertomographie

Das Produkt nach Beispiel 3 wird Patienten in einer Dosis von 2 ml/kg mit einer Injektionsgeschwindigkeit von 4 ml/sec intravenös injiziert. 25 sec nach Injektionsbeginn wird der Bereich der Leber von cranial nach caudal mit einer Schichtdicke von 0,5 cm und einem Tischvorschub von 0,7 cm/sec für einen Zeitraum von 30 sec bei Atemstillstand mit einem Spiral-CT gescannt. Es entsteht eine Darstellung des Abdomens im Bereich der Leber in 30 Schichten, in der gut durchblutete Bereiche eine erhöhte Strahlenabsorption zeigen. Auf diese Weise sind bestimmte Erkrankungen der Leber wesentlich besser zu erkennen, als ohne Kontrastmittel.

### Beispiel 5: Anwendung in der Computertomographie

30 ml des Produktes nach Beispiel 3 werden mit 970 ml Trinkwasser verdünnt. Die Lösung wird vor einer Untersuchung des Abdomens mittels der Computertomographie langsam im Laufe 1 Stunde getrunken. Magen und Darm sind durch das Kontrastmittel in der Computertomographie besser abgrenzbar.

## Patentansprüche

1. Verbindungen der allgemeinen Formel I 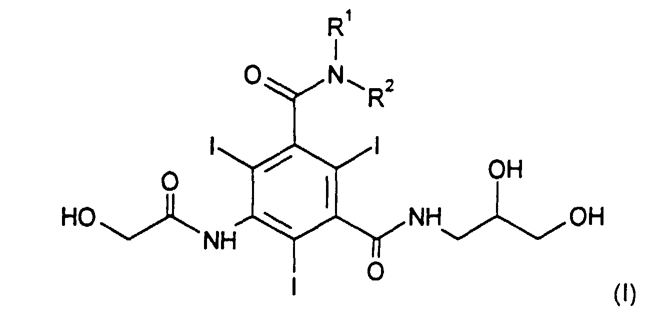 worin entweder
- R¹ für den Methylrest und R² für den 2,3-Dihydroxypropylrest steht oder
- R¹ und R² jeweils für den 2-Hydroxyethylrest stehen.

2. Verfahren zur Herstellung von Verbindungen der allgemeinen Formel I, dadurch gekennzeichnet, daß eine Verbindung der allgemeinen Formel II 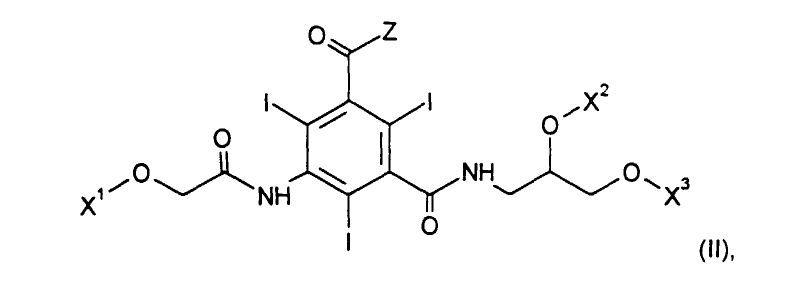 worin,
X¹, X² und X³ für Hydroxyschutzgruppen stehen und
Z für einen reaktiven Säure- oder Esterrest steht,
in einem polaren Lösungsmittel oder einem Lösungsmittelgemisch, enthaltend mindestens ein polares Lösungsmittel,
bei einer Temperatur von 0°C bis 120°C,
gegebenenfalls in Anwesenheit einer Hilfsbase
mit Diethanolamin oder N-Methylamino-propandiol umgesetzt wird und anschließend die Hydroxyschutzgruppen in bekannter Weise abgespalten werden.

3. Verfahren zur Herstellung von Verbindungen der allgemeinen Formel I, dadurch gekennzeichnet, daß eine Verbindung der allgemeinen Formel III 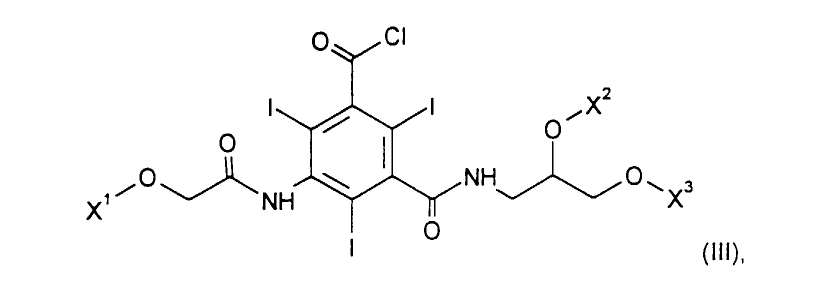 worin,
X¹, X² und X³ für Hydroxyschutzgruppen stehen
in einem polaren Lösungsmittel oder einem Lösungsmittelgemisch, enthaltend mindestens ein polares Lösungsmittel,
bei einer Temperatur von 0°C bis 120°C,
in Anwesenheit einer Hilfsbase
mit Diethanolamin oder N-Methylamino-propandiol umgesetzt wird und anschließend die Hydroxyschutzgruppen in bekannter Weise abgespalten werden.

4. Verfahren gemäß Anspruch 2 oder 3 dadurch gekennzeichnet, daß mindestens eine der Gruppen X¹, X² oder X³ für einen Acetylrest oder einen Acetoxyacetylrest steht.

5. 1 Verfahren gemäß Anspruch 2 oder 3 dadurch gekennzeichnet, daß X² und X³ gemeinsam für einen Rest der Formel 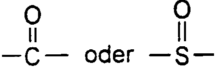 stehen.

6. Verfahren gemäß Anspruch 2 oder 3 dadurch gekennzeichnet, daß die Hilfsbase Triethylamin, Tributylamin, Natriumcarbonat oder Kaliumcarbonat ist.

7. Verfahren gemäß Anspruch 2 oder 3 dadurch gekennzeichnet, daß als Lösungsmittel Aceton, Dioxan, Dimethoxyethan, Diethylenglycoldimethylether, Dimethylacetamid oder Dimethylformamid oder Gemische dieser Lösungsmittel verwendet werden.

8. Verfahren gemäß Anspruch 2 oder 3 dadurch gekennzeichnet, daß die Umsetzung in Anwesenheit von Wasser durchgeführt wird.

9. Pharmazeutisches Mittel, enthaltend mindestens eine Verbindung der allgemeinen Formel I.

10. Pharmazeutisches Mittel gemäß Anspruch 9, enthaltend mindestens eine Verbindung der allgemeinen Formel I zusammen mit einem Calciumkomplex der Ethylendiaminpentaessigsäure.

11. Pharmazeutisches Mittel gemäß Anspruch 9, enthaltend mindestens eine Verbindung der allgemeinen Formel I zusammen mit Gefäßdilatatoren und/oder Gerinnungshemmern.

12. Verwendung von Verbindungen der allgemeinen Formel I für die Herstellung von Röntgenkontrastmitteln.

13. Verwendung von Verbindungen der allgemeinen Formel I gemäß Anspruch 12 für die Herstellung von Röntgenkontrastmitteln für die Computertomographie.

14. Verwendung von Verbindungen der allgemeinen Formel I gemäß Anspruch 12 für die Herstellung von Röntgenkontrastmitteln für die Urographie, die Myelographie und/oder die interventionelle Radiologie.

15. Verwendung von Verbindungen der allgemeinen Formel I gemäß Anspruch 12 und 13 für die Herstellung von Röntgenkontrastmitteln die interventionelle Radiologie mittels Computertomographie.

16. Verwendung von Verbindungen der allgemeinen Formel I gemäß Anspruch 12 für die Herstellung von Röntgenkontrastmitteln für diagnostische Darstellung des Magen-Darm-Traktes und/oder der Leber.

## Claims

1. Compounds of the general formula I 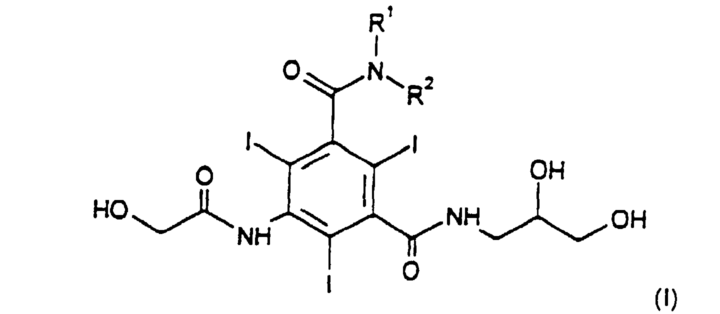 in which either
- R¹ represents the methyl radical and R² represents the 2,3-dihydroxypropyl radical or
- R¹ and R² each represent the 2-hydroxyethyl radical.

2. Process for preparing compounds of the general formula I, characterized in that a compound of the general formula II 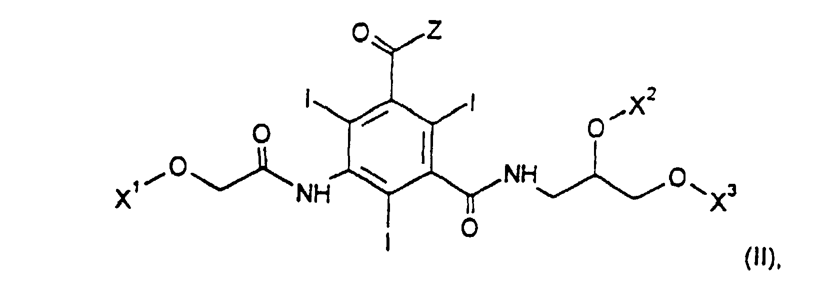 in which
X¹, X² and X³ represent hydroxyl protective groups, and Z represents a reactive acid or ester residue,
is reacted in a polar solvent or a solvent mixture containing at least one polar solvent,
at a temperature of from 0°C to 120°C,
where appropriate in the presence of a base,
with diethanolamine or N-methylaminopropanediol, and subsequently the hydroxyl protective groups are eliminated in a known manner.

3. Process for preparing compounds of the general formula I, characterized in that a compound of the general formula III 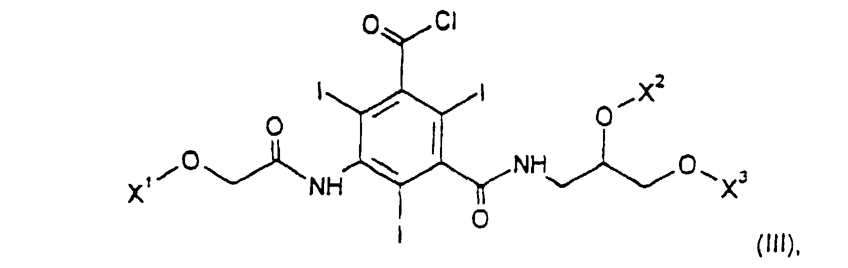 in which
X¹, X² and X³ represent hydroxyl protective groups, is reacted in a polar solvent or a solvent mixture containing at least one polar solvent,
at a temperature of from 0°C to 120°C,
in the presence of a base,
with diethanolamine or N-methylaminopropanediol, and subsequently the hydroxyl protective groups are eliminated in a known manner.

4. Process according to Claim 2 or 3, characterized in that at least one of the groups X¹, X² or X³ represents an acetyl radical or an acetoxyacetyl radical.

5. Process according to Claim 2 or 3, characterized in that X² and X³ together represent a radical of the formula

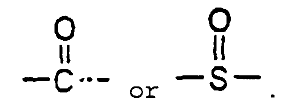

6. Process according to Claim 2 or 3, characterized in that the base is triethylamine, tributylamine, sodium carbonate or potassium carbonate.

7. Process according to Claim 2 or 3, characterized in that acetone, dioxane, dimethoxyethane, diethylene glycol dimethyl ether, dimethylacetamide or dimethylformamide or mixtures of these solvents are used as solvent.

8. Process according to Claim 2 or 3, characterized in that the reaction is carried out in the presence of water.

9. Pharmaceutical composition comprising at least one compound of the general formula I.

10. Pharmaceutical composition according to Claim 9, comprising at least one compound of the general formula I together with a calcium complex of ethylenediaminetetraacetic acid.

11. Pharmaceutical composition according to Claim 9, comprising at least one compound of the general formula I together with vasodilators and/or anticoagulants.

12. Use of compounds of the general formula I for producing X-ray contrast media.

13. Use of compounds of the general formula I according to Claim 12 for producing X-ray contrast media for computed tomography.

14. Use of compounds of the general formula I according to Claim 12 for producing X-ray contrast media for urography, myelography and/or interventional radiology.

15. Use of compounds of the general formula I according to Claim 12 and 13 for producing X-ray contrast media for interventional radiology by means of computed tomography.

16. Use of compounds of the general formula I according to Claim 12 for producing X-ray contrast media for diagnostic visualization of the gastrointestinal tract and/or of the liver.

## Revendications

1. Composés de formule générale I 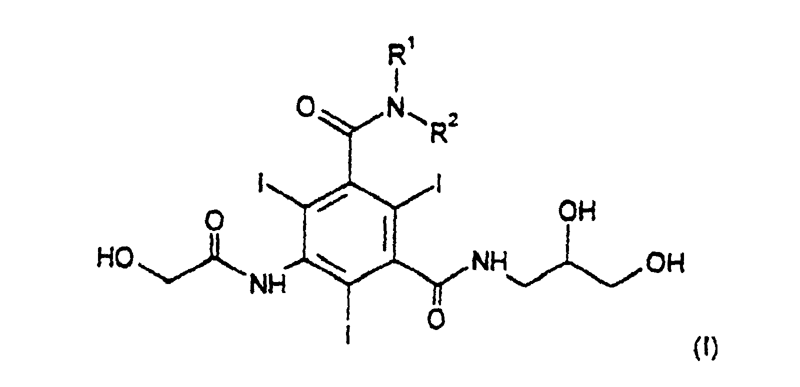 dans laquelle soit
- R¹ représente le radical méthyle et R² le radical 2,3-dihydroxypropyle
soit
- R¹ et R² représentent chaque fois le radical 2-hydroxyéthyle.

2. Procédé pour la préparation de composés de formule générale I, caractérisé en ce qu'un composé de formule générale II 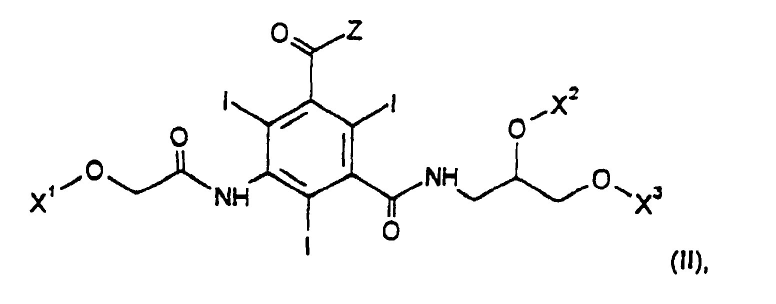 dans laquelle
X¹, X² et X³ représentent des groupements protecteurs hydroxyle, et
Z représente un radical réactif acide ou ester,
est transformé avec de la diéthanolamine ou du N-méthylaminopropandiol dans un solvant polaire ou un mélange de solvants, contenant au moins un solvant polaire,
à une température de 0°C à 120°C,
le cas échéant en présence d'une base auxiliaire et en ce qu'ensuite les groupements protecteurs hydroxyle sont séparés de manière connue.

3. Procédé pour la préparation de composés de formule générale I, caractérisé en ce qu'un composé de formule générale III 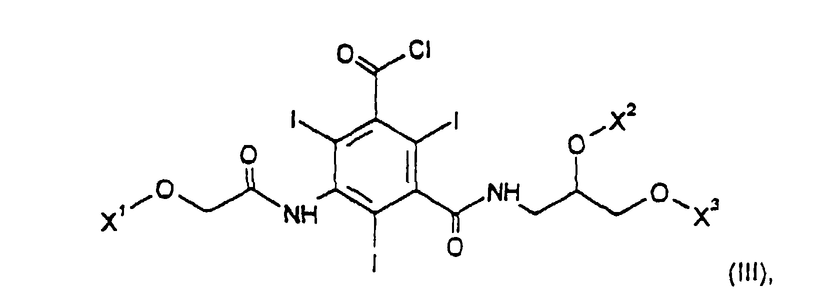 dans laquelle
X¹, X² et X³ représentent des groupements protecteurs hydroxyle
est transformé avec de la diéthanolamine ou du N-méthylaminopropandiol dans un solvant polaire ou un mélange de solvants, contenant au moins un solvant polaire,
à une température de 0°C à 120°C,
en présence d'une base auxiliaire et en ce qu'ensuite les groupements protecteurs hydroxyle sont séparés de manière connue.

4. Procédé suivant la revendication 2 ou 3, caractérisé en ce qu'au moins un des groupements X¹, X² ou X³ représente un radical acétyle ou un radical acétoxyacétyle.

5. Procédé suivant la revendication 2 ou 3, caractérisé en ce que X² et X³ représentent ensemble un radical de formule

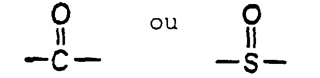

6. Procédé suivant la revendication 2 ou 3, caractérisé en ce que la base auxiliaire est de la triéthylamine, de la tributylamine, du carbonate de sodium ou du carbonate de potassium.

7. Procédé suivant la revendication 2 ou 3, caractérisé en ce qu'on utilise comme solvant de l'acétone, du dioxanne, du diméthoxyéthane, du diéthylèneglycoldiméthyléther, du diméthylacétamide ou du diméthylformamide ou des mélanges de ces solvants.

8. Procédé suivant la revendication 2 ou 3, caractérisé en ce que la transformation s'effectue en présence d'eau.

9. Produit pharmaceutique, contenant au moins un composé de formule générale I.

10. Produit pharmaceutique suivant la revendication 9, contenant au moins un composé de formule générale I conjointement avec un complexe de calcium de l'acide éthylènediaminepentacétique.

11. Produit pharmaceutique suivant la revendication 9, contenant au moins un composé de formule générale I conjointement avec des vasodilatateurs et/ou des anticoagulants.

12. Utilisation de composés de formule générale I pour la préparation de produits de contraste radiographiques.

13. Utilisation de composés de formule générale I suivant la revendication 12, pour la préparation de produits de contraste radiographiques pour la tomographie informatisée.

14. Utilisation de composés de formule générale I suivant la revendication 12, pour la préparation de produits de contraste radiographiques pour l'urographie, la myélographie et/ou la radiologie interventionnelle.

15. Utilisation de composés de formule générale I suivant la revendication 12 et 13, pour la préparation de produits de contraste radiographiques, pour la radiologie interventionnelle au moyen de la tomographie informatisée.

16. Utilisation de composés de formule générale I suivant la revendication 12, pour la préparation de produits de contraste radiographiques pour la représentation diagnostique de la partie estomac-intestin et/ou du foie.
